# EUROPÄISCHE PATENTSCHRIFT

(11) **EP 1 890 779 B1**
(45) Veröffentlichungstag und Bekanntmachung des Hinweises auf die Patenterteilung: **01.12.2010**
(21) Anmeldenummer: 06723750.3
(22) Anmeldetag: 27.03.2006
(51) Int. Cl.: A63B 71/14, A63B 69/36, A61B 5/22

(54) **GOLFTRAININGSHANDSCHUH**
GOLF TRAINING GLOVE
GANT D'ENTRAINEMENT AU GOLF

(30) Priorität: 20.04.2005 DE 102005018527
(43) Veröffentlichungstag der Anmeldung: 27.02.2008
(73) Patentinhaber: Bauer, David, 52062 Aachen (DE)
(72) Erfinder: Bauer, David, 52062 Aachen (DE)
(74) Vertreter: Kohlmann, Kai
(86) Internationale Anmeldenummer: PCT/EP2006/002772
(87) Internationale Veröffentlichungsnummer: WO 2006/111245

(56) Entgegenhaltungen:
- WO-A-00/01303
- DE-A1- 19 854 237
- DE-C1- 4 240 531
- US-A- 5 221 088
- US-A- 5 681 993
- US-A- 5 733 201
- US-A- 6 016 103

## Beschreibung

Die Erfindung betrifft einen Golftrainingshandschuh mit Sensoren zur Auslösung eines wahrnehmbaren Signals bei fehlerhafter Grifftechnik des Golfschlägers sowie mit Fingerlingen zur Aufnahme von Daumen, Zeigefinger, Mittelfinger, Ringfinger und kleinem Finger.

Derzeit existieren Trainingshilfen für Golfspieler, die im wesentlichen auf mechanische Aspekte des Golfschwunges zielen und dem Spieler das Erlernen von richtigen Positionen und Bewegungsrichtungen in bestimmten Schwungphasen erleichtern sollen. Hierbei wird jedoch ein weiterer wichtiger Aspekt vernachlässigt bzw. sogar verhindert, nämlich dass der Spieler möglichst ohne übermäßige Körperspannung schwingen sollte. Diese Anspannung hat mehrere schädliche Auswirkungen:

Angespannte Handgelenke verhindern, dass sich die im Schlägerkopf gespeicherte Bewegungsenergie nicht oder zum falschen Zeitpunkt entlädt.

Durch angespannte Unterarme wird ein gegenseitiges Rotieren der Unterarme und sogenanntes Freigeben des Schlägerkopfes blockiert, was zu verminderter Schlägerkopfgeschwindigkeit und fehlerhafter Ausrichtung des Schlägerblattes im Treffzeitpunkt führt.

Bestimmte Muskelpartien im Körper arbeiten gegeneinander und reduzieren die Schwunggeschwindigkeit indem die natürliche Bewegung des Schlägers blockiert wird.

Ein Golfspieler, der sich dieser Zusammenhänge nicht bewusst ist, wird mit dem Willen, den Ball möglichst weit schlagen zu wollen, schädliche Spannung in den Händen (und daraus folgend in den Unterarmen und anderen Körperpartien) aufbauen, und das genaue Gegenteil erreichen, nämlich zu kurze und unpräzise Schläge.

Die GB 21 20 082 A offenbart einen Golftrainingshandschuh, der automatisch ein akustisches Alarmsignal generiert, wenn sich die Griffstärke während des Golfschlages lockert. Um dieses Lockern der Griffstärke zu signalisieren, weist der Golftrainingshandschuh druckbetätigte Schalter an der Innenseite des Handschuhs auf. An jedem Finger sowie am Daumen befindet sich jeweils ein Schalter. Darüber hinaus ist an der Außenseite der Handfläche ein weiterer Schalter angeordnet. Die druckbetätigten Schalter sind vorzugsweise als Unterbrecher parallel zueinander geschaltet. Die parallel geschalteten Schalter liegen in Reihe mit einer Batterie und einem Summer. Solange der Golfgriff ausreichend fest gehalten wird, sind die zehn Unterbrecherschalter geöffnet und kein akustisches Signal wird von dem Summer erzeugt. Wird jedoch der Griff gelöst, so dass sich einer der Schalter schließt, wird das akustische Signal erzeugt.

Mit dieser Lösung lässt sich lediglich sicher stellen, dass der Golfschläger mit einer Mindestkraft festgehalten wird. Die Anordnung der Sensoren ist jedoch nicht in der Lage, unerwünschte, schädliche Spannungen in den Händen zu kontrollieren und zu vermeiden.

Aus der US 56 55 223 ist ein Golftrainingshandschuh bekannt, der wahrnehmbare Signale erzeugt, wenn der Griff während des Schwungs gelockert wird. Der Golftraingshandschuhs weist zu diesem Zweck einen druckabhängigen Sensor im Bereich der Handfläche und der Rückseite des Daumens auf. Der auf dem Ansatz des Daumens angeordnete Sensor spricht an, wenn bei dem üblichen überlappenden Griff die nachgezogene Hand gelockert wird. Der in der Handfläche angeordnete Sensor soll bei einer zu weiten Ausholbewegung ansprechen, bei der die Führungshand unbeabsichtigt gelockert wird.

Auch dieser Golftrainingshandschuh kann nicht vermeiden, dass schädliche Spannungen in den Händen aufgebaut und in Folge dessen zu kurze und unpräzise Schläge ausgeführt werden. Das Gegenteil ist vielmehr der Fall. Wird der Golfschläger mit hoher Muskelspannung, also verkrampft gehalten, kommt es auf Grund dieses Golftrainingshandschuhs keinesfalls zu einem Ansprechen der Signalgeber.

Die US 2002/0129437 A1 offenbart einen Drucksensor in dem Daumen eines Golftraingshandschuhs. Problematisch ist hierbei, dass ein Spieler leicht in der Lage ist, den Schläger mit den Fingern fest zu greifen und dennoch den Daumen entspannt oder sogar vom Griff abstehen zu lassen. Hierbei kommt der Effekt zum Tragen, dass sich der Daumen relativ unabhängig von den restlichen Fingern kontrollieren lässt. Ein weiterer Nachteil besteht darin, dass selbst bei einem völlig entspannten Griffdruck das Schlägergewicht im oberen Umkehrpunkt des Schwunges von beiden Daumen nach unten abgestützt werden muss. Dadurch, dass der Schlägerschwerpunkt deutlich vom Griff entfernt ist, wird durch Hebelwirkung die Kraft auf die Daumen verstärkt, so dass die angegebene Auslöseschwelle von 600-700g deutlich überschritten wird.

Die US2002/0194668 A1 offenbart einen Drucksensor in den Handschuh der linken Hand einzubauen, der in der oberen Handinnenfläche unterhalb der Fingeransätze liegt. Diese Idee beruht auf der Annahme, dass der Schläger bei einer korrekten Grifftechnik entlang dieser Linie anliegt und von den Fingern in den Drucksensor gedrückt wird. Ein unerfahrener Golfspieler mit einer schlechten Grifftechnik wird den Schläger nicht zuverlässig in den Bereich des Drucksensors hineindrücken. Problematisch ist außerdem, ob der Schlägergriff den Drucksensor nicht oder nur teilweise überlappt. Weitaus gravierender sind jedoch folgende Nachteile: Bei einer korrekten Schwungtechnik sollte der Schläger zu Beginn des Schwunges mit der linken Hand weggeschoben und nicht etwa mit der rechten Hand weggezogen werden. Durch das unkorrekte Wegziehen mit der rechten Hand müssen sich die Finger automatisch anspannen, um der Trägheit des Schlägers entgegenzuwirken, während die korrekte Schwungauslösung mit der linken Hand keine schädliche Spannung aufbaut, da alle Finger entspannt bleiben können. Bei der korrekten Technik zur Schwungauslösung wird jedoch durch die Trägheit des Schlägers entgegen der Bewegungsrichtung beim Aufschwung eine Kraft auf den Drucksensor in der Handinnenfläche ausgeübt, so dass selbst bei einem völlig entspanntem, korrektem Griff der Sensor auslöst.

Ein weiterer Nachteil besteht darin, dass ebenso wie bei der Platzierung des Drucksensors in dem Daumen gemäß der US 2002/0129437A1 im oberen Umkehrpunkt eine große Kraft auf den Drucksensor in der Handfläche ausgeübt wird, da die linke Handinnenfläche das Schlägergewicht nach oben abstützt. Diese kraft ist sogar noch größer als die Kraft auf die Daumen, da im Kräftegleichgewicht die einzige nach oben wirksame Kraft an dieser Stelle auftritt. Weiterhin wird bei dieser Lösung der Drucksensor lediglich in den linken Handschuh eingebaut, da aus schwungtheoretischen Gründen ein erhöhter Griffdruck in der linken Hand schädlichere Auswirkungen hat, als in der rechten Hand. Es ist jedoch wünschenswert, auch den Griffdruck der rechten Hand zu überwachen, weil ein Golfspieler problemlos in der Lage ist, bewusst oder unbewusst in beiden Händen einen unterschiedlichen Griffdruck aufzubauen.

Die US-A-5 681 993 offenbart einen Golfhandschuh aus Leder oder textilem Material zur Messung der Handkraft eines Menschen. Eine Umwandlungseinheit wandelt die Ausgangssignale von Kraftsensoren in für Menschen wahrnehmbare Signale um.

In einer Ausgestaltung des Golfhandschuhs sind zwei Kraftsensoren an der Handinnenfläche des Handschuhs an definierten Positionen angeordnet. Diese Kraftsensoren sind über ein Kabel mit einer Elektronik verbunden. In einer anderen Ausgestaltung des Golfhandschuhs wird von jedem Kraftsensor eine variable Frequenz als Ausgangssignal erzeugt. Sämtliche Sensoren sind in dem jeweils ersten Segment jedes Fingerlings des Handschuhs einschließlich demjenigen zur Aufnahme des Daumens angeordnet, wobei jeder Sensor mit einer Elektronik verbunden ist.

Die US-A-5 733 201 offenbart eine Golftrainingshilfe zur Verbesserung des Golfschwungs. Um einen zu festen Griff des Golfschlägers zu vermeiden wird ein Handschuh mit einer Vielzahl von Sensoren vorgeschlagen, die insbesondere an der Handinnenfläche und/oder an den Fingerling des Handschuhs angeordnet werden.

Ausgehend von diesem Stand der Technik liegt der Erfindung die Aufgabe zugrunde, einen Golftrainingshandschuh zu schaffen der die vorgenannten Nachteile nicht aufweist, insbesondere bei korrekter Grifftechnik keine Fehlsignalisierungen erzeugt und den Spieler dabei unterstützt, schädliche Spannung in den Händen während des Golfschwungs zu vermeiden.

Die Erfindung basiert auf dem Gedanken, dass die Finger den Griff umschließen und als Aktuatoren den eigentlichen Griffdruck aufbauen. Eine Messung an dieser Stelle liefert die am wenigsten verfälschten Resultate.

Im einzelnen wird die Aufgabe bei einem Golftrainingshandschuh der eingangs erwähnten Art dadurch gelöst, dass die Sensoren zumindest an den Fingerlingen zur Aufnahme von Zeigefinger, Mittelfinger und Ringfinger angeordnet sind und das wahrnehmbare Signal bei einem zu hohen Griffdruck auslösen und dass kein Sensor (21) an dem Daumen des Handschuhs (12,19,37) angeordnet ist.

Das wahrnehmbare Signal wird insbesondere von einer Auswerteelektronik ausgelöst, wenn sich im Rahmen eines Schwellwertvergleichs der von den Sensoren erfassten Messwerte mit mindestens einem Schwellwert ergibt, dass ein zu hoher Griffdruck aufgebaut wurde. Auf die Platzierung eines Sensors in dem Daumen wird vorzugsweise verzichtet, da insbesondere der Drucksensor in dem Daumen des Handschuhs für Fehlsignalisierungen verantwortlich ist.

Vorzugsweise werden die Drucksensoren in alle Fingerlinge außer dem Daumen in den Golftraingshandschuhs sowohl der linken als auch der rechten Hand eingebaut.

In bevorzugter Ausgestaltung der Erfindung werden die Sensoren in eine Tasche eingearbeitet, die durch eine doppelte Außenhaut an der Fingerinnenseite gebildet wird.

Als Sensortypen kommen insbesondere elektrische und pneumoelektrische Sensoren in Betracht. Als elektrischer Sensor kommt ein kapazitiver Sensor zum Einsatz, der den Vorteil hat, auch im gebogenen Zustand korrekte Ergebnisse zu liefern (im Gegensatz z.B. zum resistiven Sensor), und auch statische Kräfte erfasst (im Gegensatz zum piezoelektrischen-Sensor).

In einer kostengünstigeren Lösung kann der Griffdruck jedoch auch mit pneumolektrischen Sensoren über Druckkammern, insbesondere in Form von Luftkissen erfolgen, die in jedem Fingerling des Handschuhes in einer Tasche eingearbeitet sind. Die Druckmessung erfolgt dann über einen elektropneumatischen Wandler, der über einen Schlauch mit dem jeweiligen Kissen verbunden ist. Um nicht bereits durch die alleinige Fingerkrümmung einen Überdruck zu messen, kann es sinnvoll sein, das Kissen eines Fingerlings in 2 oder 3 Segmente aufzuteilen, die mit einem Schlauch miteinander verbunden werden.

Jedes Kissen kann zumindest teilweise von einem Profil aus elastischem Material umgeben sein, wobei die maximale Profilhöhe die Höhe des Kissens übersteigt, um das Kissen vor erhöhtem Druck zu schützen und dem Spieler einen direkteren Kontakt zum Schlägergriff zu geben. Das Profil kann in die Tasche des Fingerkissens eingebaut und beispielsweise als relativ fester Gummischlauch ausgebildet sein.

Um die Formstabilität der Kissen zu gewährleisten besteht darüber hinaus die Möglichkeit, diese teilweise mit einem elastischen Material, insbesondere Schaumstoff zu füllen. Der gasdurchlässige Schaumstoff dient dazu, die Luftkissen der pneumoelektrischen Sensoren in einer definierten Form zu halten, auch dann, wenn das Luftkissen noch nicht mit dem elektropneumatischen Wandler über eine Leitung verbunden ist. Hierdurch wird die Herstellung des erfindungsgemäßen Golftrainingshandschuhs vereinfacht.

Die Luftkissen bestehen insbesondere aus einer Kunststofffolie. Der als Leitung zur Verbindung des Luftkissens mit dem elektropneumatischen Wandler dienende Schlauch ist vorzugsweise mit dem Kissen verschweißt.

In vorteilhafter Ausgestaltung der Erfindung liefern die Sensoren analoge elektrische Signale, die in einer Auswerteelektronik nach einer Analog-/Digitalwandlung verarbeitet werden. Die Hauptaufgabe der Auswerteelektronik besteht darin, in Abhängigkeit der erfassten Messwerte an den Sensoren zu den einzelnen Fingern zu entscheiden, ob der Griffdruck einen kritischen Schwellwert überstiegen hat und dies gegebenenfalls zu signalisieren. In einer einfachen Ausgestaltung der Erfindung, wird ein einheitlicher Schwellwert für die Sensoren sämtlicher Fingerlinge festgelegt. Vorzugsweise erlaubt die Auswerteelektronik jedoch einen individuellen Schwellwertvergleich für die Messwerte jedes Sensors. Auf Grund des individuellen Schwellwertvergleichs lassen sich die bei korrekter Grifftechnik unterschiedlichen Kräfte an den einzelnen Fingern präzise berücksichtigen. Der Schwellwertvergleich mit den analogen elektrischen Signalen erlaubt darüber hinaus eine differenzierte Betrachtung der Greifkräfte für die linke und rechte Hand beim Golfschwung.

Damit kurzfristige Druckspitzen an einzelnen Sensoren, die auf Grund der Kinematik des Golfschwungs unvermeidbar oder sogar gewünscht sind, keine Fehlsignalisierung auslösen, weist die Auswerteelektronik Mittel zur zeitlichen Glättung oder Messwerte auf. Hierdurch wird sichergestellt, dass nur eine Mindestdauer anhaltende Überschreitungen des Schwellenwertes die Signalisierung auslösen. Zur zeitlichen Glättung können die Messwerte beispielsweise mit einem Tiefpass gefiltert werden. Zusätzlich oder alternativ kann die Auswerteelektronik Mittel zum Skalieren der Messwerte aufweisen. Durch die Skalierung werden die Messwerte entweder verstärkt oder gedämpft. Durch diese Maßnahme kann dem Umstand Rechnung getragen werden, dass jeder Finger bei subjektiv gleich empfundener Anspannung auf den ihm zugeordneten Sensor einen unterschiedlichen Druck ausübt. Beispielsweise ist der Zeigefinger stärker als der Ringfinger, so dass sich eine Verstärkung des Signals für den Ringfinger gegenüber dem Zeigefinger empfehlen kann. Andererseits übt der Schlägergriff zu Beginn des Schwungs einen erhöhten Druck auf den kleinen Finger aus. Damit dieser zusätzliche Druck auf den dem kleinen Finger zugeordneten Sensor nicht fälschlich als zu hoher Griffdruck interpretiert wird, empfiehlt es sich, die Messwerte dieses Sensors leicht zu dämpfen. Nimmt man den Messwert des Sensors am Zeigefinger als Bezugspunkt, hat sich eine Verstärkung der Messwerte der Sensoren an Mittel- und Ringfinger und eine Dämpfung des Messwertes des Sensors am kleinen Finger als vorteilhaft herausgestellt.

Ein Golfhandschuh ist bei regem Spielbetrieb einem starken Verschleiß ausgesetzt, so dass dieser nach einiger Zeit ersetzt werden muss. Die Auswerteelektronik des Golftrainingshandschuhs unterliegt jedoch praktisch keinem Verschleiß. In einer vorteilhaften Ausgestaltung der Erfindung ist daher die Auswerteelektronik lösbar mit dem Golftrainingshandschuh verbunden. Sie lässt sich zerstörungsfrei von dem verschlissenen Golftrainingshandschuh trennen und an einem neuen Golftrainingshandschuh anbringen. Dieser neue Golftrainingshandschuh enthält die elektrischen Sensoren oder im Falle pneumoelektrischer Sensoren, die Luftkissen einschließlich der Anschlussleitungen an den elektropneumatischen Wandler. Die elektropneumatischen Wandler sind vorzugsweise ebenfalls lösbar mit dem Golftrainingshandschuh verbunden und können bei dessen Austausch weiterverwendet werden.

Die Verbindung der lösbaren Auswerteelektronik erfolgt über eine elektrische Kopplung zu den Zuleitungen zu den elektrischen Sensoren oder durch eine Kopplung der Luftschläuche an die elektropneumatischen Wandler.

Fehlsignalisierungen durch das auf die Hand einwirkende Schlägergewicht werden reduziert, wenn die Fingerlinge des Handschuhs in drei Segmente unterteilt sind, wobei die Sensoren in dem ersten oder dem ersten und zweiten Segment, ausgehend von der Spitze jedes Fingerlings, angeordnet sind. Dabei wird davon ausgegangen, dass das erste Segment jedes Fingerlings zur Aufnahme des distalen, das zweite Segment jedes Fingerlings zur Aufnahme des medialen und das dritte Segment jedes Fingerlings zur Aufnahme des proximalen Glieds des Fingers dient.

Um weitgehend unabhängig von einer idealen Griffhaltung bei geringst möglicher Anzahl der Sensoren Fehlsignalisierungen auf Grund falscher Messwerte zu vermeiden, sind in den Fingerlingen zur Aufnahme des kleinen Fingers, des Ringfingers sowie des Mittelfingers die Sensoren in dem ersten Segment, ausgehend von der Spitze jedes Fingerlings, angeordnet und in dem Fingerling zur Aufnahme des Zeigefingers ist der Sensor in dem zweiten Segment, ausgehend von der Spitze des Fingerlings, angeordnet. Insbesondere bei pneumoelektrischen Sensoren hat sich herausgestellt, dass bei dieser Anordnung der Sensoren der Druck für die unterschiedlichsten Griffhaltungen am zuverlässigsten gemessen wird und die Sensoren vom Spieler als nicht störend wahrgenommen werden. Die Luftkisten der pneumoelektrischen Sensoren des kleinen Fingers, des Ringfingers sowie des Mittelfingers befinden sich möglichst nah am Übergang zum medialen Segment, d. h. in der Nähe des Gelenkes zum medialen Fingerglied. Für den Zeigefinger ist die Platzierung des Luftkissens des elektropneumatischen Sensors am medialen Segment, d. h. im Bereich des mittleren medialen Fingergliedes, vorteilhaft, weil der Zeigefinger den Schlägergriff weniger als die übrigen Finger umschließt, so dass im Bereich des medialen Fingerglieds der größte Druck vom Zeigefinger ausgeübt wird. Außerdem wird durch diese Platzierung automatisch der vom gegenüberliegenden Daumen ausgeübte Druck mitgemessen, da dieser bei einigermaßen korrekter Griffhaltung entgegengesetzt zum medialen Fingerglied des Zeigefingers auf der gegenüberliegenden Seite des Schlägergriffs aufliegt. Gleichzeitig lässt sich durch diese bevorzugte Anordnung der Luftkissen sicherstellen, dass die Luftkissen der Sensoren vollflächig belastet werden. Bei nur teilweiser Komprimierung der Luftkissen, wird nur ein Teil der Luft verdrängt und in Folge dessen ein falscher Messwert erfasst.

In einer weiteren Ausgestaltung der Erfindung weist die Auswerteelektronik Mittel zum drahtlosen Übertragen von Daten, insbesondere der in der Auswerteelektronik verarbeiteten Messwerte auf. Die Datenübertragung erlaubt die Protokollierung sowie die weitere Verarbeitung der Messwerte mit einem Personal Computer sowie die Sichtbarmachung von Fehlern bei der Greiftechnik auf dessen Anzeigeeinheit.

Die Erfindung wird nachfolgend anhand der Figuren näher erläutert. Es zeigen:
- Figur 1: die Kraftverteilung im oberen Umkehrpunkt eines Golfschwunges;
- Figur 2: die Darstellung einer korrekten und inkorrekten Schwungauslösung;
- Figur 3: ein erstes Ausführungsbeispiel eines erfindungsgemäßen Golftrainingshandschuhs;
- Figur 4: ein zweites Ausführungsbeispiel eines erfindungsgemäßen Golftraingshandschuhs ;
- Figur 5: ein prinzipielles Blockschaltbild einer Auswertelektronik;
- Figur 6: ein drittes Ausführungsbeispiel eines erfindungsgemäßen Golftraingshandschuhs sowie
- Figur 7: eine Seitenansicht einer lösbar mit dem Golftraingshandschuhs verbindbaren Auswerteelektronik.

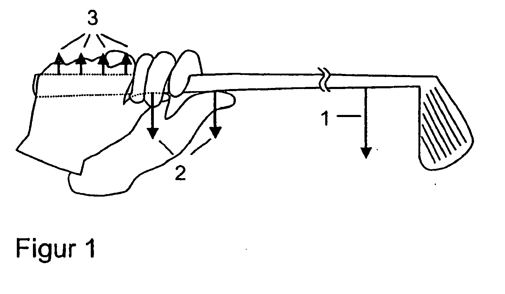

Figur 1 zeigt die Kraftverteilung im oberen Umkehrpunkt des Schwunges, wobei Pfeil 1 die Gewichtskraft des Schlägers im Schwerpunkt, die Pfeile 2 die Unterstützungskraft der Daumen und die Peile 3 die Kraft auf die linke Handinnenfläche symbolisieren.

Figur 1 verdeutlicht dass selbst bei einem völlig entspannten Griffdruck das Schlägergewicht im oberen Umkehrpunkt des Schwunges von beiden Daumen nach unten abgestützt werden muss. Dadurch, dass der Schlägerschwerpunkt deutlich vom Griff entfernt ist, wird durch Hebelwirkung die Kraft auf die Daumen verstärkt. Die Anordnung eines Sensors im Daumen führt deshalb zu Fehlsignalisierungen.

Figur 1 verdeutlicht weiter dass im oberen Umkehrpunkt des Schwungs eine große Kraft auf die Handinnenfläche ausgeübt wird, da die linke Handinnenfläche das Schlägergewicht nach oben abstützt. Diese Kraft ist sogar noch größer als die Kraft auf die Daumen, da im Kräftegleichgewicht die einzige nach oben wirksame Kraft an dieser Stelle auftritt. Die Anordnung eines Sensors in der Handinnenfläche führt daher ebenfalls zu Fehlsignalisierungen im Umkehrpunkt des Schwungs.

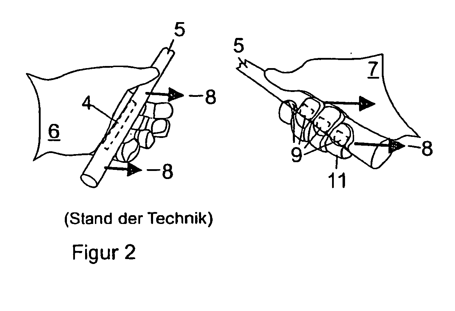

Weitaus gravierender sind jedoch folgende Nachteile eines Sensors 4 in der Handinnenfläche eines Golftraingshandschuhs, die anhand von Figur 2 erläutert werden:

Bei einer korrekten Schwungtechnik sollte der Schläger 5 zu Beginn des Schwunges mit der linken Hand 6 weggeschoben und nicht etwa mit der rechten Hand 7 weggezogen werden. Durch das unkorrekte Wegziehen mit der rechten Hand 7 spannen sich die Finger automatisch an, um der Trägheit des Schlägers 5 entgegenzuwirken, während die korrekte Schwungauslösung mit der linken Hand 6 keine schädliche Spannung aufbaut, da alle Finger entspannt bleiben können. Bei der korrekten Technik zur Schwungauslösung wird jedoch durch die Trägheit des Schlägers entgegen der durch die Pfeile 8 gekennzeichneten Bewegungsrichtung beim Aufschwung eine Kraft auf den Sensor 4 in der Handinnenfläche ausgeübt, so dass selbst bei einem völlig entspanntem, korrektem Griff der Sensor 4 auslöst. Werden hingegen erfindungsgemäß Sensoren 9 in die Fingerlinge 11 der rechten Hand 7 eingebaut, löst die falsche Schwungauslösetechnik diese Sensoren 9 aus, während die richtige Schwungtechnik die Sensoren nicht auslösen würde.

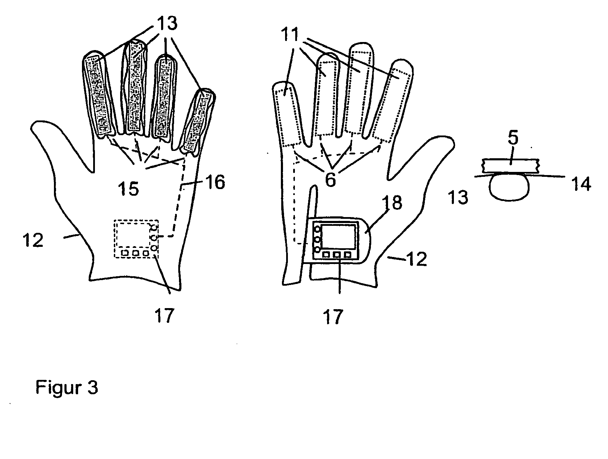

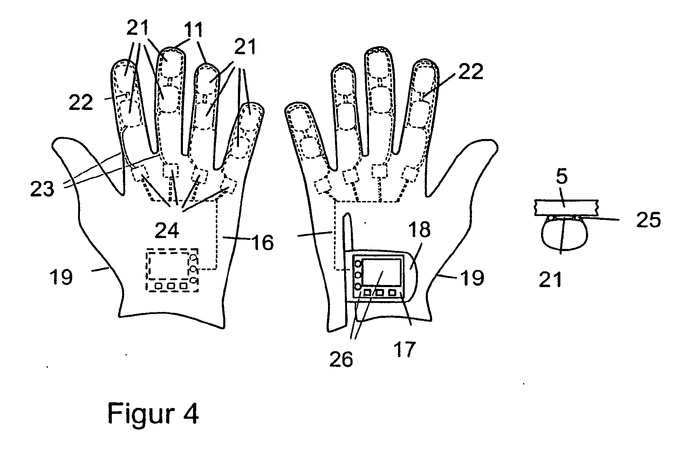

Der prinzipielle Aufbau eines erfindungsgemäßen Golftrainingshandschuhs ist für unterschiedliche Sensortechnologien in den Figuren 3 und 4 für einen linken Handschuh 12 dargestellt.

Figur 3 zeigt einen erfindungsgemäßen Handschuh 12 mit elektrischen Sensoren 13, die an der Innenseite der Fingerlinge 11 über deren gesamte Länge angeordnet sind. Die Sensoren 13 befinden sich in Taschen, deren Außenhaut 14 rechts im Bild angedeutet ist. Die Anschlüsse 15 der Sensoren 13 stehen über Verbindungsleitungen 16 mit einer Auswerteelektronik 17 in Verbindung, die in einem Verschluss 18 für den Handschuh 12 auf dessen Oberseite untergebracht ist.

Figur 4 zeigt einen Handschuh 19 bei dem sich Luftkissen 21 pneumoelektrischer Sensoren 21, 24 nur in den ersten beiden Segmenten der Fingerlinge 11 befinden. Ein elastischer Wulst 25 umgibt die Luftkissen 21. Der Schläger 5 liegt auf dem Wulst 25 und den Luftkissen 21 auf. Die Luftkissen 21 sind, die beiden vorderen Segmente jedes Fingerlings überbrückend, mit Schläuchen 22 miteinander verbunden. Jeweils ein weiterer Schlauch 23 verbindet jeweils jedes Luftkissenpaar in einem Fingerling 11 mit einem elektropneumatischen Wandler 24, wobei jeder Wandler 24 wiederum über elektrische Verbindungsleitungen 16 mit der Auswertelektronik 17 im Handschuhverschluss 18 verbunden ist.

Für die Sensoren des in Figur 4 dargestellten Handschuhs 12 der linken Hand 6 gilt, dass alle Finger (außer dem Daumen) den Griff gleichmäßig umschließen und somit gleich gewichtet werden können.

Werden aus Kostengründen nur kleine Sensoren, d. h. solche mit nur einem Luftkissen je Sensor, eingesetzt, sollten diese in den oberen Segmenten der Fingerlinge 11 liegen. Für die Sensoren des Handschuhs der rechten Hand 7 gilt, dass aus Kostengründen auf einen Sensor im Fingerling 11 für den kleinen Finger verzichtet werden kann, da in den allermeisten Grifftechniken dieser nicht am Schläger anliegt.

Unabhängig von der verwendeten Technik liefern die Sensoren 21,24 für jeden Finger einen analogen Messwert, der in der Auswerteelektronik 17 nach einer Analog-/Digital Wandlung durch einen digitalen Mikrocontroller weiterverarbeitet wird. Aufgabe dieser Auswerteelektronik 17 ist es, in Abhängigkeit der ermittelten Druckwerte der einzelnen Finger zu entscheiden, ob der Griffdruck eine kritischen Wert überstiegen hat und dies durch Licht, Ton oder Vibration zu signalisieren.

Die Frage, ob ein Alarm ausgelöst werden sollte, hängt von dem Schwellenwert sowie von der Dauer der Überschreitung dieses Wertes ab. Es ist möglich, für jeden einzelnen Finger ein gesonderten Schwellenwert festzulegen. In jedem Fall ist zumindest ein gemeinsamer Schwellenwert für sämtliche Sensoren 21, 24 in den Fingerlingen 11 stufenlos oder in Stufen einstellbar, um bestimmte Schwierigkeitsgrade einzustellen (Anfänger, Fortgeschrittener). Außerdem kann eine Unterscheidung des Schwellenwertes zwischen dem Handschuh 12 für die linke und rechte Hand 6,7 vorgesehen werden.

Damit zufällige Messfehler (Druckspitzen) und nur kurzzeitige, aber gewünschte Überschreitungen (z.B. im Zeitpunkt des Ball- bzw. Bodenkontaktes) keine Fehlsignalisierung auslösen, sollen nur längerfristige Überschreitungen des Schwellenwertes die Signalisierung auslösen. Hierzu können die gemessenen Sensorwerte z.B. mit einem Tiefpass gefiltert werden.

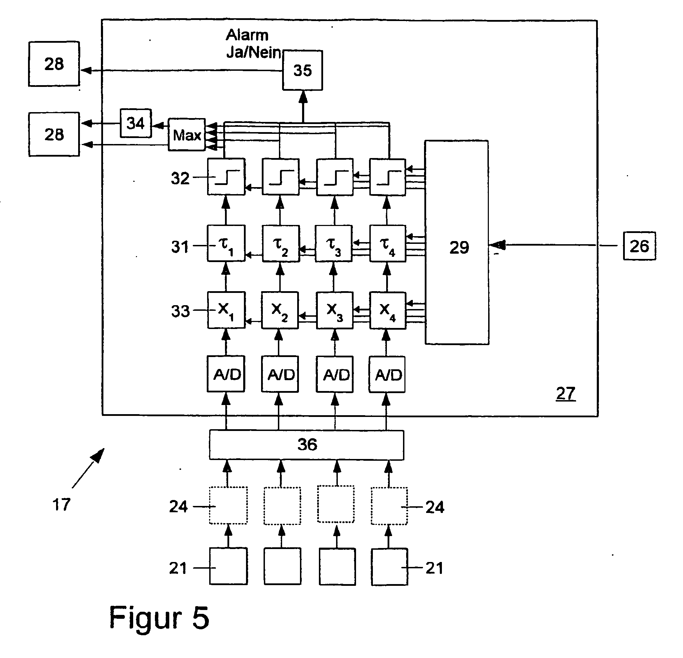

Das prinzipielle Blockschaltbild einer Auswerteelektronik 17 ist in Figur 5 dargestellt. Ein insgesamt mit 27 bezeichneter Mikrocontroller dient der Messwertverarbeitung sowie Koordination der Ein- und Ausgabeelemente 26,28. Die Eingaben erfolgen über in den Figuren 3, 4 und 6 erkennbare Tasten, während die Ausgabe über optische und akustische Signalisierungsmittel 28, wie beispielsweise ein Display oder Lautsprecher erfolgen.

Über die Tasten der Auswerteelektronik 17 wird ein Benutzerprofil in einen Speicher 29, beispielsweise ein "Look-Up-Table" eingegeben. Das Benutzerprofil umfasst individuelle Schwellwerte für die von jedem Sensor 21,24 erfassten Messwerte, sowie Werte zur Skalierung und zeitlichen Glättung der Messwerte jedes Sensors 21,24. Der Schwellwertvergleich der Messwerte der Sensoren 21,24 erfolgt in Komparatoren 32 für den Signalweg jedes einzelnen Sensors. In dem Signalweg befinden sich darüber hinaus Tiefpassfilter 31 für die zeitliche Glättung sowie Dämpfungs- bzw. Verstärkungselemente 33 für die Skalierung. Stellt einer der Komparatoren 32 fest, dass der Messwert den für das jeweilige Benutzerprofil vorgegebenen Schwellwert überschreitet, wird dies für jeden Signalweg mit den Signalisierungsmitteln 28 angezeigt. Dem akustischen Signalisierungsmittel 28 ist ein spannungsgesteuerter Oszillator 34 vorgeschaltet. Der spannungsgesteuerte Oszillator 34 erzeugt abhängig von dem vom Komparator 32 erzeugten Ausgangssignal unterschiedlich frequente Ausgangsspannungen, die beispielsweise in einem Lautsprecher unterschiedlich hohe Töne erzeugen. Je nach Größe der Schwellwertüberschreitung werden daher unterschiedlich akustische Signale erzeugt.

Darüber sind die Ausgänge sämtlicher Komparatoren 32 mit einer Oder-Schaltung 35 verbunden. Diese löst ebenfalls über die weiteren optischen/akustischen Signalisierungsmittel 28 einen Alarm aus, wenn lediglich einer der Komparatoren 32 eine Schwellwertüberschreitung feststellt.

Auf der Eingangsseite weist die Auswerteelektronik 17 für jeden Messkanal einen Analog-/Digitalwandler A/D auf, der die analogen Messsignale aus dem pneumoelektrischen Wandler 24 hinter einer Signalaufbereitung 36 in digitale Signale wandelt.

Als Skalierungsfaktoren X1 - X4 für die Dämpfungs- bzw. Verstärkungselemente 33 sind beispielsweise folgende Faktoren praktikabel, wobei X1 den Messwerten des Sensors des Zeigefingers, X2 den Messwerten des Sensors des Mittelfingers, X3 den Messwerten des Sensors des Ringfingers und X4 den Messwerten des Sensors des kleinen Fingers zugeordnet ist:
X1 = 1,0
X2 = 1,0 - 1,2
X3 = 1, 0 - 1,25
X4 = 0,8 - 0,95

Die Dämpfung des kleinen Fingers ist zweckmäßig, da der Schlägergriff bei der Schwungauslösung einen verstärkten Druck auf den kleinen Finger auslöst. Damit dieser zusätzliche Druck auf den kleinen Finger nicht fälschlicherweise als zu hoher Griffdruck von dem Komparator 32 erfasst wird, wird der Messwert am kleinen Finger gedämpft, während die Messwerte an den gegenüber dem Zeigefinger schwächeren Mittel- und Ringfingern gegenüber dem Zeigefinger geringfügig verstärkt werden.

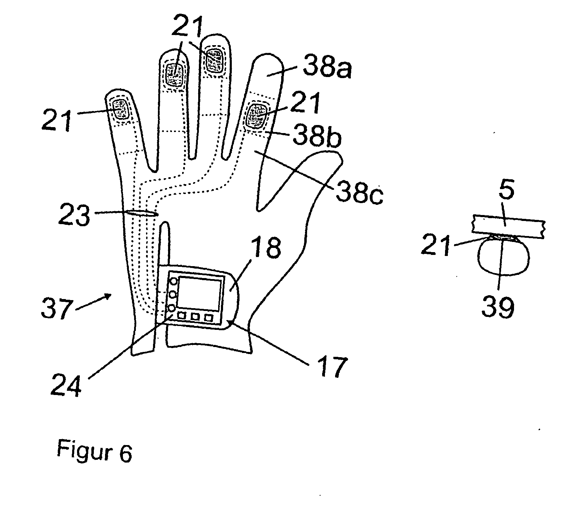

Figur 6 zeigt einen Handschuh 37 bei dem die elektropneumatischen Sensoren 21, 24 so angeordnet sind, dass der vom Spieler auf den Griff ausgeübte Druck weitgehend unabhängig von einer idealen Griffhaltung erfasst werden kann. Die Fingerlinge 11 des Handschuhs 37 sind in drei Segmente 38 a - c unterteilt, wobei in den Fingerlingen zur Aufnahme des kleinen Fingers, des Ringfingers sowie des Mittelfingers die Luftkissen 21 der Sensoren 21,24 in dem ersten Segment 38 a angeordnet sind und in dem Fingerling zur Aufnahme des Zeigefingers das Luftkissen 21 in dem zweiten Segment 38 b angeordnet ist. Die einzelnen Luftkissen 21 stehen über Schläuche 23 mit den Wandlern 24 in Verbindung. Die pneumoelektrischen Wandler 24 liefern analoge Signale, die nach der Analog-/Digitalwandlung in der Auswerteelektronik 17 nach Figur 5 weiter verarbeitet werden.

Die Luftkissen 21 sind mit Schaumstoff 39 gefüllt, der die Luftkissen 21 in einer definierten Form hält. Die Auswerteelektronik 17 ist auch bei diesem Handschuh 37 in den Verschluss 18 integriert und weist übereinstimmend Ein- und Ausgabeelemente 26,28 auf.

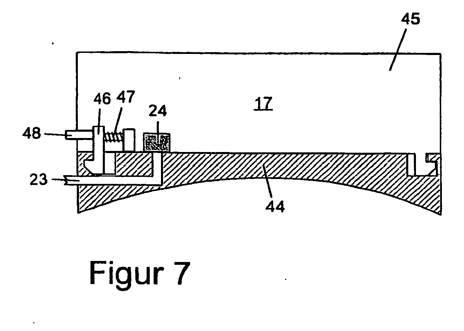

Figur 7 zeigt schließlich eine Seitenansicht einer Auswerteelektronik 17, die lösbar mit dem Handschuh 12, 19 bzw. 37 verbunden ist. Zu diesem Zweck wird ein, der Kontur des Handrückens angepasster Sockel 44 an der Oberseite des Handschuhs 12,19, 37 befestigt. An dem Sockel 44 wird das, die Auswerteelektronik 17 aufnehmende Gehäuse 45 lösbar verhakt. Hierzu ist mindestens einer der Haken 46 gegen die Kraft einer Feder 47 beweglich ausgeführt. Durch betätigen eines sich durch die Wand des Gehäuses 45 erstreckenden Stiftes 48, lässt sich der bewegliche Haken 46 verschieben, so dass das Gehäuse 45 freigegeben wird.

Die Kopplung der pneumoelektrischen Wandler 24 mit den Schlauchleitungen 23 erfolgt dadurch, dass die Endstücke der Schlauchleitungen in entsprechende Aufnahmen der Wandler 24 eingreifen. Bei elektrischen Sensoren in den Fingerlingen münden die von den Sensoren führenden elektrischen Leitungen in Kontaktflächen an der Oberfläche des Sockels 44, die mit entsprechenden Kontaktflächen an der Unterseite des Gehäuses 45 zusammenwirken.

Die abnehmbare Auswerteelektronik 17, lässt sich weiter verwenden, wenn der Handschuh verschlissen ist. Lediglich die relativ preiswerten Luftkissen 21 einschließlich der daran angeschweißten Schläuche 23 werden ausgetauscht.

### Bezugszeichenliste:

| Nr. | **Bezeichnung** | Nr. | **Bezeichnung** |
|---|---|---|---|
| 1. | Gewichtskraft Schläger | 29. | Speicher |
| 2. | Unterstützungskraft Daumen | 30. | - |
| 3. | Kraft auf Handinnenfläche | 31. | Tiefpassfilter |
| 9. | Sensor Handinnenfläche | 32. | Komparatoren |
| 5. | Schläger | 33. | Dämpfungs-/Verstärkungselemente |
| 6. | Linke Hand | 34. | Spannungsgesteuerter Oszillator |
| 7. | Rechte Hand | 35. | Oder-Schaltung |
| 8. | Bewegungsrichtung Aufschwung | 36. | Signalaufbereitung |
| 9. | Sensor Fingerling | 37. | Handschuh |
| 10. | - | 38 a) - c) | Segmente |
| 11. | Fingerlinge | 39. | Schaumstoff |
| 12. | Handschuh | 40. | - |
| 13. | Sensor | 41. | Tasten |
| 14. | Außenhaut | 42. | Benutzerprofil |
| 15. | Anschluss | 43. | Anzeige |
| 16. | Verbindungsleitung | 44. | Sockel |
| 17. | Auswerteelektronik | 45. | Gehäuse |
| 18. | Verschluss | 46. | Haken |
| 19. | Handschuh | 47. | Feder |
| 20. | - | 48. | Stift |
| 21. | Luftkissen | | |
| 22. | Schlauch | | |
| 23. | Schlauch | | |
| 24. | Wandler | | |
| 25. | Wulst | | |
| 26. | Ein-/Ausgabeelement | | |
| 27. | Mikrocontroller | | |
| 28. | Opt./akust. Signalisierungsmittel | | |

## Patentansprüche

1. Golftrainings-Handschuh mit Sensoren zur Auslösung eines wahrnehmbaren Signals bei fehlerhafter Grifftechnik des Golfschlägers mit Fingerlinge zur Aufnahme von Daumen, Zeigefinger, Mittelfinger, Ringfinger und kleinem Finger, **dadurch gekennzeichnet, dass** die Sensoren (13,21,24) zumindest an den Fingerlingen (11) zur Aufnahme von Zeigefinger, Mittelfinger und Ringfinger angeordnet sind und das wahrnehmbare Signal bei einem zu hohen Griffdruck auslösen und dass kein Sensor (21) an dem Daumen des Handschuhs (12,19,37) angeordnet ist.

2. Golftrainingshandschuh nach Anspruch 1, **dadurch gekennzeichnet, dass** ein weiterer Sensor (13,21,24) an dem Fingerling (11) für den kleinen Fingern angeordnet ist.

3. Golftrainingshandschuh nach Anspruch 1 oder 2, **dadurch gekennzeichnet, dass** die Sensoren (13,21,24) an der Innenseite der Fingerlinge (11) angeordnet sind.

4. Golftrainingshandschuh nach einem der Ansprüche 1 bis 3, **dadurch gekennzeichnet, dass** die Sensoren (13,21,24) in Tauschen (14) eingearbeitet sind.

5. Golftrainingshandschuh nach einem der Ansprüche 1 bis 4, **dadurch gekennzeichnet, dass** die Sensoren als elektrische Sensoren (13) ausgebildet sind.

6. Golftrainingshandschuh nach Anspruch 5, **dadurch gekennzeichnet, dass** die elektrischen Sensoren(13) kapazitive Sensoren sind.

7. Golftrainingshandschuh nach einem der Ansprüche 1 bis 4, **dadurch gekennzeichnet, dass** die Sensoren als pneumoelektrische Sensoren (21,24) ausgebildet sind.

8. Golftrainingshandschuh nach Anspruch 7, **dadurch gekennzeichnet, dass** die pneumoelektrischen Sensoren (21,24) mehrere durch Leitungen(22,23) miteinander verbundene Druckkammern (21) aufweisen.

9. Golftrainingshandschuh nach Anspruch 7 oder 8, **dadurch gekennzeichnet, dass** jede Druckkammer (21) des pneumoelektrischen Sensors über eine Leitung (23) mit einem elektropneumatischen wandler (24) verbunden ist, der die Druckschwankungen in jeder Druckkammer (21) in analoge elektrischen Signale umsetzt.

10. Golftrainingshandschuh nach einem der Ansprüche 7 -9,
**dadurch gekennzeichnet**, das jede Druckkammer (21) des pneumoelektrischen Sensor (21,24) mit einem elastischen, gasdurchlässigen Material(39) zumindest teilweise gefüllt ist.

11. Golftrainingshandschuh nach einem der Ansprüche 1 bis 10, **dadurch gekennzeichnet, dass** die von den Sensoren erfassten Messwerte in analoge elektrische Signale umgesetzt werden, die in einer Auswerteelektronik nach einer Analog-/Digitalwandlung verarbeitet werden.

12. Golftrainingshandschuh nach Anspruch 11, **dadurch gekennzeichnet, dass** die Auswerteelektronik (17) einen individuellen Schwellwertvergleich für die Messwerte jedes Sensors erlaubt.

13. Golftrainingshandschuh nach Anspruch 11 oder 12, **dadurch gekennzeichnet, dass** die Auswerteelektronik Mittel zum Skalieren (33) und /oder zur zeitlichen Glättung (31) der Messwerte aufweist.

14. Golftrainingshandschuh nach Anspruch 13, **dadurch gekennzeichnet, dass** die Auswerteelektronik (17) Mittel (33) zum getrennten Skalieren und /oder zur getrennten zeitlichen Glättung (31) jedes Messwertes aufweist.

15. Golftrainingshandschuh nach einem der Ansprüche 11 bis 14, **dadurch gekennzeichnet, dass** die Auswerteelektronik (17,45) lösbar mit dem Golftrainingshandschuh (12,19,37) verbunden ist.

16. Golftrainingshandschuh nach Anspruch 11 und 15, **dadurch gekennzeichnet, dass** die Auswertelektronik (17) einschließlich aller elektropneumatischen wandler (24) lösbar mit dem Golftrainingshandschuh (19,37) verbunden sind.

17. Golftrainingshandschuh nach einem der Ansprüche 1 bis 16, **dadurch gekennzeichnet, dass** die Fingerlinge (11) des Handschuhs (12) in drei Segmente unterteilt sind, wobei die Sensoren in dem ersten oder dem ersten und zweiten Segment (38a,38b) ausgehend von der Spitze jedes Fingerlings (11) angeordnet sind.

18. Golftrainingshandschuh nach Anspruch 8 und 17, **dadurch gekennzeichnet, dass** eine erste Druckkammer (21) im Bereich des ersten Segmentes (38a) und eine zweite Druckkammer (21) im Bereich des zweiten Segmentes (38b) angeordnet ist.

19. Golftrainingshandschuh nach einem der Ansprüche 1 bis 16, **dadurch gekennzeichnet, dass** die Fingerlinge (11) des Handschuhs (12) in drei Segmente (38a, 38b, 38c) unterteilt sind, wobei in den Fingerlingen zur Aufnahme des kleinen Fingers, des Ringfingers sowie des Mittelfingers die Sensoren (9) oder die Druckkammern (21) der Sensoren (21,24) in dem ersten Segment (38a) ausgehend von der Spitze jedes Fingerlings (11) angeordnet sind und in dem Fingerling zur Aufnahme des Zeigefingers der Sensor (9) oder die Druckammer (21) in dem zweiten Segment (38b) ausgehend von der Spitze des Fingerlings (11) angeordnet ist.

20. Golftrainingshandschuh nach einem der Ansprüche 17 - 19, **dadurch gekennzeichnet, dass** das erste Segment (38a) jedes Fingerlings (11) zur Aufnahme des distalen, das zweite Segment (38b) jedes Fingerlings zur Aufnahme des medialen und das dritte Segment (38c) jedes Fingerlings zur Ausnahme des proximalen Glieds des Fingers dient.

21. Golftrainingshandschuh nach einem der Anspruche 8 bis 10, **dadurch gekennzeichnet, dass** jede Druckkammer (21) zumindest teilweise von einem Profil (25) aus elastischem Material umgeben ist, wobei die maximale Profilhöhe die Höhe der Druckkammer (21) übersteigt.

22. Golftrainingshandschuh nach einem der Ansprüche 11 bis 21, **dadurch gekennzeichnet, dass** die Auswertelektronik (17) Mittel zur drahtlosen Übertragen von Daten aufweist.

## Claims

1. A golf training glove with sensors for triggering a perceptible signal in the event of an incorrect gripping technique of the golf club, with finger stalls for receiving thumb, index finger, middle finger, ring finger and little finger, **characterised in that** the sensors (13, 21, 24) are disposed at least on the finger stalls (11) for receiving index finger, middle finger and ring finger, and trigger the perceptible signal if a gripping pressure is too high and that no sensor (21) is disposed on the thumb of the glove (12, 19, 37).

2. The golf training glove according to claim 1, **characterised in that** another sensor (13, 21, 24) is disposed on the finger stall (11) for the little finger.

3. The golf training glove according to claim 1 or 2, **characterised in that** the sensors (13, 21, 24) are disposed on the inner side of the finger stalls (11).

4. The golf training glove according to any one of claims 1 to 3, **characterised in that** the sensors (13, 21, 24) are incorporated in pockets (14).

5. The golf training glove according to any one of claims 1 to 4, **characterised in that** the sensors are configured as electrical sensors (13).

6. The golf training glove according to claim 5, **characterised in that** the electrical sensors (13) are capacitive sensors.

7. The golf training glove according to any one of claims 1 to 4, **characterised in that** the sensors are configured as pneumo-electric sensors (21, 24).

8. The golf training glove according to claim 7, **characterised in that** the pneumo-electric sensors (21, 24) comprise a plurality of pressure chambers (21) interconnected by lines (22, 23).

9. The golf training glove according to claim 7 or 8, **characterised in that** each pressure chamber (21) of the pneumo-electric sensor is connected via a line (23) to an electro-pneumatic converter (24) which converts the pressure fluctuations in each pressure chamber (21) into analogue electric signals.

10. The golf training glove according to any one of claims 7-9, **characterised in that** each pressure chamber (21) of the pneumo-electric sensor (21, 24) is at least partially filled with an elastic, gas-permeable material (39).

11. The golf training glove according to any one of claims 1 to 10, **characterised in that** the measured values detected by the sensors are converted into analogue electric signals which are processed in an electronic evaluation system following analogue/digital conversion.

12. The golf training glove according to claim 11, **characterised in that** the electronic evaluation system (17) allows an individual threshold value comparison for the measured values of each sensor.

13. The golf training glove according to claim 11 or 12, **characterised in that** the electronic evaluation system has means for scaling (33) and/or temporally smoothing (31) the measured values.

14. The golf training glove according to claim 13, **characterised in that** the electronic evaluation system (17) has means (33) for the separate scaling and/or the separate temporal smoothing (31) of each measured value.

15. The golf training glove according to any one of claims 11 to 14, **characterised in that** the electronic evaluation system (17, 45) is detachably connected to the golf training glove (12, 19, 37).

16. The golf training glove according to claim 11 and 15, **characterised in that** the electronic evaluation system (17) including all the electro-pneumatic converters (24) is detachably connected to the golf training glove (19, 37).

17. The golf training glove according to any one of claims 1 to 16, **characterised in that** the finger stalls (11) of the glove (12) are divided into three segments, wherein the sensors are disposed in the first or the first and second segment (33a, 38b) starting from the tip of each finger stall (11).

18. The golf training glove according to claim 3 and 17, **characterised in that** a first pressure chamber (21) is disposed in the region of the first segment (38a) and a second pressure chamber (21) is disposed in the region of the second segment (38b).

19. The golf training glove according to any one of claims 1 to 16, **characterised in that** the finger stalls (11) of the glove (12) are divided into three segments (38a, 38b, 38c), wherein in the finger stalls for receiving the small finger, the ring finger and the middle finger, the sensors (9) or the pressure clambers (21) of the sensors (21, 24) are disposed in the first segment (38a) starting from the tip of each finger stall (11) and in the finger stall for receiving the index finger, the sensor (9) or the pressure chamber (21) is disposed in the second segment (38b) starting from the tip of the finger stall (11).

20. The golf training glove according to any one of claims 17-19, **characterised in that** the first segment (38a) of each finger stall (11) serves to receive the distal member, the second segment (38b) of each finger stall serves to receive the medial member and the third segment (38c) of each finger stall serves to receive the proximal member of the finger.

21. The golf training glove according to any one of claims 8 to 10, **characterised in that** each pressure chamber (21) is at least partially surrounded by a profile (25) of elastic material, wherein the maximum profile height exceeds the height of the pressure chamber (21).

22. The golf training glove according to any one of claims 11 to 21, **characterised in that** the electronic evaluation system (17) comprises means for wireless transmission of data.

## Revendications

1. Gant d'entraînement pour le golf muni de capteurs pour le déclenchement d'un signal perceptible dans le cas d'une technique de préhension incorrecte du club de golf, avec des doigtiers recevant le pouce, l'index, le majeur, l'annulaire et l'auriculaire, **caractérisé en ce que** les capteurs (13, 21, 24) sont disposés au moins sur les doigtiers (11) recevant l'index, le majeur et l'annulaire et déclenchent le signal perceptible dans le cas d'une pression de serrage trop forte, et **en ce qu'**il n'y a pas de capteur (21) disposé sur le pouce du gant (12, 19, 37).

2. Gant d'entraînement pour le golf selon la revendication 1, **caractérisé en ce qu'**un capteur (13, 21, 24) supplémentaire est disposé sur le doigtier (11) de l'auriculaire.

3. Gant d'entraînement pour le golf selon la revendication 1 ou 2, **caractérisé en ce que** les capteurs (13, 21, 24) sont disposés sur le côté intérieur des doigtiers (11).

4. Gant d'entraînement pour le golf selon l'une des revendications 1 à 3, **caractérisé en ce que** les capteurs (13, 21, 24) sont incorporés dans des poches (14) .

5. Gant d'entraînement pour le golf selon l'une des revendications 1 à 4, **caractérisé en ce que** les capteurs sont réalisés sous la forme de capteurs électriques (13).

6. Gant d'entraînement pour le golf selon la revendication 5, **caractérisé en ce que** les capteurs électriques (13) sont des capteurs capacitifs.

7. Gant d'entraînement pour le golf selon l'une des revendications 1 à 4, **caractérisé en ce que** les capteurs sont réalisés sous la forme de capteurs pneumo-électriques (21, 24).

8. Gant d'entraînement pour le golf selon la revendication 7, **caractérisé en ce que** les capteurs pneumo-électriques (21, 24) sont munis de plusieurs chambres de compression (21) reliées entre elles au moyen de conduites (22, 23).

9. Gant d'entraînement pour le golf selon la revendication 7 ou 8, **caractérisé en ce que** chaque chambre de compression (21) du capteur pneumo-électrique est reliée, au moyen d'une conduite (23), à un convertisseur électropneumatique (24) qui convertit les variations de pression de chaque chambre de compression (21) en signaux électriques analogiques.

10. Gant d'entraînement pour le golf selon l'une des revendications 7 à 9, **caractérisé en ce que** chaque chambre de compression (21) du capteur pneumo-électrique (21, 24) est remplie, au moins en partie, d'une matière élastique (39) perméable aux gaz.

11. Gant d'entraînement pour le golf selon l'une des revendications 1 à 10, **caractérisé en ce que** les valeurs de mesure acquises par les capteurs sont converties en signaux électriques analogiques, qui sont retraités dans une électronique d'exploitation après une conversion analogique/digitale.

12. Gant d'entraînement pour le golf selon la revendication 11, **caractérisé en ce que** l'électronique d'exploitation (17) permet une comparaison individuelle des valeurs de seuil pour les valeurs de mesure de chaque capteur.

13. Gant d'entraînement pour le golf selon la revendication 11 ou 12, **caractérisé en ce que** l'électronique d'exploitation comporte des moyens de mise à l'échelle (33) et de lissage temporel (31) des valeurs de mesure.

14. Gant d'entraînement pour le golf selon la revendication 13, **caractérisé en ce que** l'électronique d'exploitation (17) comporte des moyens de mise à l'échelle individuelle (33) et de lissage temporel individuel (31) de chaque valeur de mesure.

15. Gant d'entraînement pour le golf selon l'une des revendications 11 à 14, **caractérisé en ce que** l'électronique d'exploitation (17, 45) est reliée de manière amovible avec le gant d'entraînement pour le golf (12, 19, 37).

16. Gant d'entraînement pour le golf selon les revendications 11 et 15, **caractérisé en ce que** l'électronique d'exploitation (17), y compris tous les convertisseurs électropneumatiques (24), est reliée de manière amovible avec le gant d'entraînement pour le golf (19, 37).

17. Gant d'entraînement pour le golf selon l'une des revendications 1 à 16, **caractérisé en ce que** les doigtiers (11) du gant (12) sont divisés en trois segments, les capteurs étant disposés dans le premier ou dans le premier et le deuxième segment (38a, 38b) à partir de l'extrémité de chaque doigtier (11).

18. Gant d'entraînement pour le golf selon les revendications 8 et 17, **caractérisé en ce qu'**une première chambre de compression (21) est disposée dans la zone du premier segment (38a) et une seconde chambre de compression (21) est disposée dans la zone du deuxième segment (38b).

19. Gant d'entraînement pour le golf selon l'une des revendications 1 à 16, **caractérisé en ce que** les doigtiers (11) du gant (12) sont divisés en trois segments (38a, 38b, 33c), les capteurs (9) ou les chambres de compression (21) des capteurs (21, 24) étant disposés, dans les doigtiers recevant l'auriculaire, l'annulaire et le majeur, dans le premier segment (38a) à partir de l'extrémité de chaque doigtier (11), et le capteur (9) ou la chambre de compression (21) étant disposé, dans le doigtier recevant l'index, dans le deuxième segment (38b) à partir de l'extrémité de chaque doigtier (11).

20. Gant d'entraînement pour le golf selon l'une des revendications 17 à 19, **caractérisé en ce que** le premier segment (38a) de chaque doigtier (11) reçoit la phalange distale, le deuxième segment (38b) la phalange médiale et le troisième segment (38c) la phalange proximale du doigt.

21. Gant d'entraînement pour le golf selon l'une des revendications 8 à 10, **caractérisé en ce que** chaque chambre de compression (21) est entourée au moins en partie par un profilé (25) en une matière élastique, la hauteur maximale du profité étant supérieure à la hauteur de la chambre de compression (21).

22. Gant d'entraînement pour le golf selon l'une des revendications 11 à 21, **caractérisé en ce que** l'électronique d'exploitation (17) comporte des moyens pour la transmission de données sans fil.
